# EUROPÄISCHE PATENTANMELDUNG

(11) **EP 1 496 114 A1**
(43) Veröffentlichungstag der Anmeldung: **12.01.2005**
(21) Anmeldenummer: 03015324.1
(22) Anmeldetag: 07.07.2003
(51) Int. Cl.: C12N 13/00

(54) **Verfahren zur Inaktivierung von Mikroorganismen**

(71) Anmelder: Margraf, Stefan, Dr.med., 60318 Frankfurt (DE)
(72) Erfinder: Margraf, Stefan, Dr.med., 60318 Frankfurt (DE)
(74) Vertreter: Strehl Schübel-Hopf & Partner

(57) **Zusammenfassung**

Die vorliegende Erfindung stellt ein Verfahren zur Inaktivierung eines Mikroorganismus bereit, welches das direkte Kontaktieren eines flüssigen Mediums, das den Mikroorganismus enthält, mit einer UV-Strahlung emittierenden Lichtquelle umfaßt.

## Beschreibung

### Gebiet der Erfindung

Die vorliegende Erfindung betrifft ein Verfahren zur Inaktivierung von Mikroorganismen, inaktivierte Mikroorganismen, die nach diesem Verfahren erhältlich sind, und Impfstoffe, die auf diese Weise inaktivierte Mikroorganismen enthalten.

### Hintergrund der Erfindung

Im Stand der Technik sind verschiedene Verfahren zur Inaktivierung von Mikroorganismen bekannt. Die Inaktivierung von beispielsweise Viren ist im Rahmen therapeutischer Maßnahmen von viralen Infektionen, zur Sicherstellung der Infektionsfreiheit von Blutprodukten sowie zur Impfstoff-Herstellung notwendig. Darüber hinaus ist die Virusinaktivierung eine wichtige Maßnahme aus dem Bereich der Hygiene zur Flächen-, Luft- und Abwasserentkeimung.

Nur für wenige Viruskrankheiten existiert die Option einer, meist sehr teuren, pharmakologischen Therapie. Zur Bekämpfung von hochvirämischen Virusinfektionen mit hoher Letalität (hämorrhagische Fieber, Gelbfieber, Marburgvirus, Ebola etc.) gibt es auch heute noch fast keine Therapiemöglichkeiten.

Eine Möglichkeit der Inaktivierung von Viren ist die Bestrahlung mit UV-Strahlen. Eine bekannte Anwendung von UV-Strahlen ist die Desinfektion von Raumluft mit UVC-Strahlung, wobei gewöhnlich 253,7 nm (eine Entladungslinie von Hg) zum Einsatz kommt (Berg-Perier et al. (1992) *Ultraviolet radiation and ultra-clean air enclosures in operating rooms. UV-protection, economy and comfort;* J. Arthroplasty, 7, 457-463). UVC-Strahlung zerstört DNA und RNA und löst darüberhinaus chemische Reaktionen aus, die zur Proteinzerstörung und zur Bildung von Ozon führen. Die Photonenenergie ist hoch genug, um Moleküle zu ionisieren und zu zerstören. UVC-Strahlung wirkt damit stark zelltoxisch und mutagen. Durch weitgehende Absorption der Strahlung durch biologische Materialien, Flüssigkeiten und auch Kunststoffmaterialien hat UVC nur eine geringe Eindringtiefe und ist damit nur effektiv in Luft und proteinarmen Wasser.

Bei der Impfstoffherstellung mittels Bestrahlung kommt es ganz entscheidend darauf an, dass jedes Partikel mit grösstmöglicher Sicherheit eine vorbestimmte Strahlungsdosis allseits erhält, womit die Inaktivierungskinetik präzise kalkulierbar wird. Darüberhinaus dürfen keine weiteren Destruktionen ausgelöst werden, um die antigenen Eigenschaften vollständig zu erhalten. Das ist bisher technisch sehr schwierig gewesen, weshalb bislang noch kein Impfstoff auf diese Weise hergestellt wurde.

Zur Rettung von letal bedrohten Patienten mit akuten, hochvirämischen Infektionen im Sinne einer Virusabreicherung im peripheren Blut durch UV-Blutwäsche kommt es darauf an, mit größter Effektivität die Viren zu inaktivieren bei gleichzeitiger Schonung der Blutbestanteile.

Eine universelle Methode zur Inaktivierung von Mikroorganismen, insbesondere der Virusabreicherung, wäre also wünschenswert.

### Durch die Erfindung zu lösende Aufgaben

Es ist daher eine Aufgabe der vorliegenden Erfindung, ein Verfahren bereitzustellen, mit dem Mikroorganismen in einem flüssigen Medium effektiv, berechenbar und auf einfache Weise inaktiviert werden können.
Eine weitere Aufgabe ist es, die Inaktivierung auf eine Weise durchzuführen, die es erlaubt, gezielt RNA und/oder DNA ohne Beeinträchtigung anderer Zellbestandteile oder Inhaltsstoffe zu inaktivieren.

### Gegenstand der Erfindung

Die erste Aufgabe wird durch ein Verfahren gelöst, welches das direkte Kontaktieren eines flüssigen Mediums, das den Mikroorganismus enthält, mit einer UV-Strahlung emittierenden Lichtquelle umfaßt.
Die zweite Aufgabe wird dadurch gelöst, daß bei der direkten Kontaktierung des Mikroorganismus mit der Lichtquelle UV-Licht aus einem bestimmten Wellenlängenbereich verwendet wird. Bevorzugte Wellenlängenbereiche sind 290 bis 295 nm, 285 bis 300 nm, 280 bis 305 nm, 275 bis 305 nm und 270 bis 305 nm. Insbesondere bevorzugt ist der Wellenlängenbereich, bei dem DNA/RNA-Moleküle am stärksten absorbieren bei gleichzeitig noch vertretbarer Absorption durch andere Proteine, Aminosäuren etc., nämlich 280 bis 300 nm.

### Genaue Beschreibung der Erfindung

Der Begriff Mikroorganismus umfaßt prokaryontische Mikroorganismen, wie Bakterien, eukaryontische Mikroorganismen, wie Algen, Pilze, Protozoen, aber auch höher entwickelte Zellen, wie menschliche, tierische oder pflanzliche Zellen, sowie Viren und Plasmide.

Die Intensität der eingesetzten UV-Strahlung ist vorzugsweise mindestens 1 %, stärker bevorzugt 10 % und noch stärker bevorzugt 50 % der Gesamtintensität der Strahlung.

Die UV-Strahlung wird nach DIN 5031 wie folgt unterteilt: UVA (315-380 nm), UVB (280-315 nm) und UVC (100-280 nm). Die obere Grenze des UV-Spektrums wird bei 400 nm angesetzt. Am kurzwelligen Ende geht sie allmählich in die weiche Röntgenstrahlung über.

UVA hat eine relativ lange Wellenlänge, dabei aber eine vergleichsweise hohe Eindringtiefe. Ohne photoaktive Substanzen entfaltet UVA relativ geringe Auswirkung auf biologische Materialien. Im wesentlichen beruhen die Schädigungen durch UVA-Strahlung auf der Entstehung von Sauerstoffradikalen. UVA-Strahlung kann dadurch lokal und entfernt vom Ort der Strahlung den Bruch chemischer Bindungen herbeiführen und das zelluläre Erbgut schädigen.

UVB-Strahlung hat eine höhere Potenz, biologische Schädigung hervorzurufen, da das Energie-Niveau ausreichend hoch ist, um kovalente Bindungen aufzulösen. Die Wirkung der UVB-Strahlung ist zu einem hohen Anteil darauf zurückzuführen, dass die Strahlungsenergie von Nukleinsäure absorbiert wird und dadurch schädigende Reaktionen induziert werden. Die Art der Schädigungen ist überwiegend gekennzeichnet durch Bildung von Cyclobutan-Pyrimidin-Dimeren (CPD), die durch kovalente Bindung von benachbarten Pyrimidinen einer Polynucleotid-Kette entstehen. Ferner ist unter anderem die Bildung von Pyrimidin-Pyrimidon-Läsionen (6-4 Läsionen) von Bedeutung. Zusätzlich kommt es zu Kreuzvernetzungen (Crosslinking) von DNA, aber besonders bei RNA. UVB-Strahlung wird in der Dermatologie, z.B. bei Psoriasis, therapeutisch eingesetzt.

UVC-Strahlung ist die aggressivste UV-Strahlung. Durch weitgehende Absorption der Strahlung durch biologische Materialien, Flüssigkeiten und auch Kunststoffmaterialien hat sie nur eine geringe Eindringtiefe. Das sogenannte Vakuum-UVC (100-200 nm) wird bereits durch Luft absorbiert. UVC-Strahlung kann organische Moleküle zerstören sowie ionisieren. Das große Zerstörungspotential der UVC-Strahlung erklärt sich durch die hohe Photonenenergie der kurzen Wellenlängen, so dass durch diese Strahlung beispielsweise Proteine zerstört werden.

Die Effektivität und Sicherheit einer Inaktivierung mittels UV-Strahlung hängt von verschiedenen Parametern ab.

Ist die zu inaktivierende Flüssigkeit in einer handelsüblichen Polystyrol-Zellkulturflasche eingeschlossen, dann kommt es zu einer Durchlassschwächung der Strahlungsintensität durch eine Kunststoffschicht und zu einem Reflektionsverlust. Außerdem entsteht eine Spektralerschiebung durch ein unterschiedliches, wellenlängenabhängiges Absorptionsverhalten eines jeden Werkstoffes, so dass sich im Falle von Polystyrol eine Verschiebung zugunsten der Transmission in Richtung der längerwelligen Strahlung ergibt.

Beispielsweise ist eine Strahlungsminderung von 55% bzw. 57% nachweisbar, wenn Zellkulturflaschen der Firma Greiner oder Falcon verwendet werden und die Inaktivierungskinetik ist nicht mehr linear.

Angestrebtes Ziel ist, daß die Strahlungsverteilung an jedem Punkt der Flüssigkeit möglichst gleich sein sollte, um ein homogenes Strahlungsfeld zu erzeugen. Jede Distanzierung und jede von der Strahlung zu durchdringende Materialschicht zwischen Strahlungsemittent und Ziel gefährdet die Inaktivierungssicherheit. Auch bei UV-durchlässigen Materialien kommt es zu Reflektionsverlusten sowohl am Eintritts- wie auch am Austrittsort der Strahlung. Weiterhin führt eine wie auch immer geartete Trennlage zur Distanzierung zwischen Strahlenemissionspunkt und Strahlenziel, welches eine Strahlungsminderung proportional zum Abstandsquadrat zur Folge hat. Wichtig ist auch, daß als Folge einer solchen Distanzierung Strahlen vorwiegend primär im rechten Winkel und damit nur einseitig auf das Ziel treffen. Zusätzlich führen Unreinheiten, Einschlüsse, Wandickenschwankungen und Krümmungen zu Abschattungen, Linseneffekten und Streuungen. Dies hat zur Folge, daß kein homogenes Strahlungsfeld erreicht wird und damit die Inaktivierungseffektivität, Berechenbarkeit und damit die Produktsicherheit beeinträchtigt wird.

Aus den obengenannten Gründen wird in dem erfindungsgemäßen Verfahren die zu bestrahlende Flüssigkeit direkt mit der Lichtquelle kontaktiert, so daß kein Intensitätsverlust und keine Spektralverschiebung auftreten sowie das Ziel nicht nur im 90° Winkel, sondern auch in nennenswerterweise gleichzeitig seitlich getroffen wird.

Die UV-Strahlung emittierende Lichtquelle ist vorzugsweise eine Leuchtstofflampe, wobei die zu behandelnde Flüssigkeit bevorzugt in einem Ringspalt über die Oberfläche der Lichtquelle geleitet wird. Vorzugsweise ist die Aussenhülle um die Leuchtstofflampe, die damit den Ringspalt bildet, aus Polytetrafluorethylene (PTFE), Quarzglas oder einem anderen, für UV-Strahlen durchlässigen Material. Um die Aussenhülle kann eine UV-reflektierende Schicht aufgebracht sein. Beispielsweise kann die Aussenhülle mit Aluminium bedampft oder mit poliertem Aluminium, Titan oder einer Legierung umhüllt sein.

Das Verfahren kann weiter optimiert werden, indem eine turbulente Strömung in dem zu behandelnden Medium bewirkt wird. Diese wird dadurch erzielt, daß in geeigneter Weise das Medium, vorzugsweise die Flüssigkeit, quer oder längs zur Fliessrichtung in Schwingungen versetzt wird. Zum Beispiel kann die Aussenhülle mit einer Schwingmasse oder einer unwuchtig rotierenden Masse oder einer schwingenden, mit der Flüssigkeit in Kontakt stehenden Membran (Piezo) oder mit einem Kolben verbunden sein, welcher mechanisch oder elektrisch zum Schwingen gebracht wird.

Zur Überprüfung der Leistungsfähigkeit der Virusinaktivierung durch UVB-Strahlung mit PL-S9W/12 Lampen (Wellenlänge von 290 bis 320 nm) wurden verschiedene Virussuspensionen (Adeno-3,HCMV (HI91), HSV-1, HSV-2, Coxsackie B 1, Reovirus-3, Polio-1, Echo-11, HIV-1 (RF), Corona )in zellfreiem Medium erfolgreich durch Bestrahlung vollständig inaktiviert. Die zu unterschiedlichen Zeitpunkten entnommenen Proben wurden in Verdünnungsreihen zur Infektion von permissiven Zellen eingesetzt, und die Restinfektiosität ist durch Zählung von Antigen-exprimierenden Zellen oder cytopathischen Effekten bestimmt worden. Es zeigte sich, daß die Inaktivierungskinetik im halblogarithmischen Maßstab auch bei Inaktivierung von Ausgangstitern von bis zu 1 x 10⁹ PFU/ml linear ist. Weiterhin zeigte sich, daß zur Inaktivierung besonders umweltresistenter Viren keine höheren Strahlungsdosen notwendig waren sowie die Unterschiede in den Bestrahlungsdosen für die einzelnen Virenarten gering sind (Ausnahme: 10 fach erhöhte Empfindlichkeit für Coronavirus). Eine maximale Dosis von 2,5 J/cm² war ausreichend, um Adenovirus mit dem höchsten Test-Titer (1 x 10⁹ PFU/ml) vollständig zu inaktivieren. Reaktivierungen durch Licht-induzierbare Photolyasen wurden nicht beobachtet. Bei Bestrahlung einer Virussuspension durch eine Polystyrolschicht zeigten die gewonnenen Daten im Vergleich eine stark abgeschwächte Wirkung sowie einen nichtlinearen Verlauf.

Die Ergebnisse dieser Referenzversuche zeigen, daß die Wirksamkeit einer Strahlung vermindert wird, wenn sie eine Polystyrolschicht durchdringen muß. Folglich ist das erfindungsgemäße Verfahren, bei dem es zu einem direkten Kontakt zwischen Lichtquelle und zu bestrahlender Flüssigkeit kommt, gegenüber den herkömmlichen Verfahren vorteilhaft.

Mit Hilfe des erfindungsgemäßen Verfahrens ist es möglich, DNA- oder RNA-haltige biologische Agenzien in wässrigen Lösungen bzw. Suspensionen, Blutplasma und sogar Blut zu inaktivieren.

Das erfindungsgemäße Verfahren zur Inaktivierung von Mikroorganismen kann beispielsweise zur Herstellung von Impfstoffen eingesetzt werden.

Die Inaktivierung von Viren, Parasiten, Sporen oder Bakterien in Wasser oder wässrigen Lösungen mit bestimmten Wellenlängen aus dem UV-Bereich ist besonders schonend und effektiv. Weitere bedeutsame Einsatzbereiche sind Keimabreicherung von Getränken aller Art, insbesondere Säften. Auch in der pharmazeutischen Industrie bietet die Erfindung eine neue Option zur schonenden Sterilisation von Produkten unter Einsparung nachteiliger Konservierungsmittel zum Beispiel von Produkten für die Anwendung am Auge. Milch kann besonders schonend mit dieser Anwendung haltbar gemacht werden.

Trotz Schwächung der genannten Strahlenbereiche durch Absorption an Proteinen ist die Durchdringung einer komplexen Flüssigkeit wie Blut bei weitem grösser als mit kurzwelligem UVC. Die schonende Inaktivierung mit bestimmten Strahlen des UVB-Bereichs schädigt Blut in einem sehr viel geringerem Ausmaß als dies mit UVC-Strahlen möglich wäre. Damit lässt sich durch UVB-Bestrahlung von infiziertem Blut oder Blutplasma eine Keim- bzw. Virenlast signifikant senken. Eine solche Virenlast kann zum Beispiel bei Erregern der hämorrhagischen Fieber leicht 10¹⁰ infektiöse Viren/ml und mehr betragen. Mit einer kontinuierlichen UVB-Phototherapie von Blut oder Blutplasma kann daher ein lebensgefährlich, akut an einer durch Mikroorganismen verursachten Krankheit erkrankter Patient erstmals therapiert werden, falls sich die Mikroorganismen zu einem grossen Anteil im Blut befinden. Damit entsteht die Chance den Krankheitsverlauf abzuschwächen, so daß der Organismus eine Immunantwort bilden und gesunden kann. Die Neuerung des Einsatzes von UV-Strahlung der benannten Bereiche bietet den Vorteil einer höheren Eindringtiefe bei spezifischer DNA- und RNA-Blockierung unter Schonung der Proteine sowie die direkte Kontaktierung des Ziels mit dem Strahlungsemittenten in Dünnfilmtechnik. Die Wirkung der Strahlung ist darauf zurückzuführen, dass die Strahlungsenergie von Nukleinsäure absorbiert wird und dadurch schädigende Reaktionen induziert werden.

Der Organismus profitiert auf dreierlei Weise von der Inaktivierung von Mikroorganismen infolge der bezeichneten UV-Strahlung:
A)direkter viruzider bzw. Mikroorganismen abtötender Effekt und damit Senkung der Last an infektiösen Viren bzw. Mikroorganismen
B)Impfeffekt durch präzise Übereinstimmung der Antigene des durch UV-Bestrahlung erzeugten Tot-Impfstoffes mit denen der Erreger, auch unter Berücksichtigung unterschiedlicher Erregerpopulationen mit Antigenshift durch Hypermutation
C)Auf Immunzellen des Blutes begrenzte, transiente Immunsuppression zur Unterdrückung schädlicher, überschießender Immunreaktionen, wie z. B. bei Sepsis.

Darüberhinaus bietet die Erfindung die Möglichkeit, alle im peripheren Blut vorhandenen lebenden Zellen zu inaktivieren, um einen schnelle, schonende und transiente Immunsuppresion zu erzeugen. Diese Anwendung ist von Bedeutung bei überschießender Immunreaktion wie bei Allergien, Autoimmunkrankheiten, Transplantatabstossungen, Infektionen und Operationen unter Einsatz von Herz-Lungenmaschinen und ähnlichem.

Um komplette Antigene für wissenschafliche oder medizinische Zwecke aus Viren, Parasiten, Bakterien oder Tumorzellen herzustellen, sind die genannten Strahlungsbereiche besonders gut geeignet, da die antigene Struktur unverändert erhalten bleibt. Damit lassen sich nur mit Hilfe von UV-Strahlung inaktivierte Antigene von höchster Qualität herstellen. So ist eine maximale Immunantwort bei hoher Inaktivierungssicherheit erzielbar. Das ist von großem Wert zur Stimulation von Immunzellen bei der Krebsbekämpfung und zur Herstellung von Totimpfstoffen, oder auch zur externen Stimulation von Immunzellen, z. B. zur Messung der zellulären Immunantwort. Gerade bei den Impfstoffen gibt es immer wieder Enttäuschungen wegen unzureichender Immunantworten, da die herkömmlichen Verfahren die Antigenität der zu inaktivierenden Ausgangsmaterialien stark verändern und damit beschädigen. Deshalb werden solche schlechten Impfstoffe relativ hoch dosiert, um eine Wirkung zu erzielen. Andererseits wird damit aber auch eine erhöhte Rate an Nebenwirkungen erzeugt. Für eine Reihe von Krankheiten ließ sich mit den herkömmlichen Verfahren bisher überhaupt kein funktionierender Impfstoff zum breiten Einsatz erzeugen (humanes Cytomegalievirus, Denguefieber etc.).

Zur Herstellung von Thrombozytenpräparaten wird unter anderem die PUVA-Methode evaluiert, die jedoch den Nachteil hat, daß toxisches, carzinogenes Psoralen hinzugefügt und nach Bestrahlung mit der ansonsten unwirksamen UVA-Strahlung wieder entfernt werden muss. Insbesondere für Kriseneinsätze ist es erforderlich, vor Ort zur Rettung Verletzter schnell sichere Thrombozytenkonzentrate herstellen zu können. Durch Einsatz von UV-Strahlung ist diese Aufgabe lösbar, denn nur Strahlung der genannten Bereiche kombiniert mit der erfindungsgemässen Technik sind in der Lage die empfindlichen Thrombozyten sicher zu sterilisieren ohne sie zu beschädigen und damit zu entwerten.

Was für Thrombozyten gilt, ist in besonderem Maße auch für Immunglobuline entscheidend. Diese wertvollen und unersetzbaren Medikamente sind als (gepoolte) Naturprodukte gleichermaßen empfindlich wie auch potentiell infektiös (Hepatitis A-D, HIV, etc.). Auch hier lässt sich eine Sterilisation ohne Qualitätsverlust mit Hilfe der bezeichneten UV-Strahlung erzielen.

Eine weitere wichtige Anwendung ist die Inaktivierung von Erregern tödlicher Krankheiten in Muttermilch. Die Infektion von Neugeborenen mit dem Humanen Cytomegalovirus (HCMV), Humanen Immundefizienz Virus Typ 1 (HIV-1) und Humanem T-Zell-Leukämie Virus Typ 1 (HTLV-1) geschieht größtenteils durch orale Aufnahme infizierter Muttermilch. Durch eine Sterilisation mit den bezeichneten UV-Strahlen ist es möglich, wichtige Enzymaktivitäten der Milch, im Gegensatz zum Abkochen, zu erhalten und damit die Milch bei Erhaltung der Qualität sicher zu machen. Daneben ist auch hier ein gewisser Impfeffekt von weiterem Vorteil. Bisherige Inaktivierungsverfahren wie Erwärmung oder Einfrieren sind unsicher, da selbst durch Kochen DNA nicht zerstört wird und nackte DNA infektiös ist.

Beim Purgen, d. h. beim Abtöten von Tumorzellen unter Erhalt der gesunden Zellen zur Herstellung von Knochenmarkspräparaten ist ebenso der Einsatz von der bezeichneten UV-Strahlung mit oder ohne Einsatz unterstützender Medikamente vorteilhaft. Bei begrenzter Fähigkeit von Tumorzellen, DNA-Reparaturen durchzuführen, sind diese früher irreparabel geschädigt als gesunde Zellen.

## Patentansprüche

1. Verfahren zur Inaktivierung eines Mikroorganismus, welches das direkte Kontaktieren eines flüssigen Mediums, das den Mikroorganismus enthält, mit einer UV-Strahlung emittierenden Lichtquelle umfaßt.

2. Verfahren nach Anspruch 1, wobei die Intensität der UV-Strahlung mindestens 1 % der Gesamtintensität der Strahlung beträgt.

3. Verfahren nach Anspruch 1, wobei die Intensität der UV-Strahlung mindestens 10 % der Gesamtintensität der Strahlung beträgt.

4. Verfahren nach Anspruch 1, wobei die Intensität der UV-Strahlung mindestens 50 % der Gesamtintensität der Strahlung beträgt.

5. Verfahren nach einem der vorstehenden Ansprüche, wobei die Strahlung im Wellenlängenbereich von 290 bis 295 nm liegt.

6. Verfahren nach einem der Ansprüche 1 bis 4, wobei die Strahlung im Wellenlängenbereich von 285 bis 300 nm liegt.

7. Verfahren nach einem der Ansprüche 1 bis 4, wobei die Strahlung im Wellenlängenbereich von 280 bis 305 nm liegt.

8. Verfahren nach einem der Ansprüche 1 bis 4, wobei die Strahlung im Wellenlängenbereich von 275 bis 305 nm liegt.

9. Verfahren nach einem der Ansprüche 1 bis 4, wobei die Strahlung im Wellenlängenbereich von 270 bis 295 nm liegt.

10. Verfahren nach einem der Ansprüche 1 bis 4, wobei die Strahlung im Wellenlängenbereich von 290 bis 310 nm liegt.

11. Verfahren nach einem der vorstehenden Ansprüche, wobei der Mikroorganismus unter Viren, Parasiten, Sporen, Bakterien und Tumorzellen ausgewählt ist.

12. Verfahren nach einem der vorstehenden Ansprüche, wobei der Mikroorganismus in Wasser, einer wäßrigen Lösung, einer nichtwässrigen Flüssigkeit, Blut, verdünntem Blutplasma, Thrombozytenpräparaten, Luft, Immunglobulinpräparaten, Erythrozytenkonzentraten oder Muttermilch mit der Lichtquelle kontaktiert wird.

13. Inaktivierter Mikroorganismus, erhältlich durch das Verfahren nach einem der Ansprüche 1 bis 12.

14. Impfstoff, der den inaktivierten Mikroorganismus nach Anspruch 13 enthält.
